# EUROPEAN PATENT SPECIFICATION

(11) **EP 1 742 587 B1**
(45) Date of publication and mention of the grant of the patent: **13.07.2011**
(21) Application number: 05737050.4
(22) Date of filing: 21.04.2005
(51) Int. Cl.: A61B 17/92

(54) **DEVICE AND METHOD FOR INSERTING, POSITIONING AND REMOVING AN IMPLANT**
VORRICHTUNG UND VERFAHREN ZUM EINFÜHREN, POSITIONIEREN UND ENTFERNEN EINES IMPLANTATS
DISPOSITIF ET PROCEDE PERMETTANT D'INTRODUIRE, DE POSITIONNER ET DE RETIRER UN IMPLANT

(30) Priority: 23.04.2004 US 831569
(43) Date of publication of application: 17.01.2007
(73) Proprietor: Synthes GmbH, 4436 Oberdorf (CH)
(72) Inventor: KMIEC, Stanley, J., Jr., Morgantown, PA 19543 (US)
(74) Representative: Höhfeld, Jochen
(86) International application number: PCT/US2005/013600
(87) International publication number: WO 2005/104968

(56) References cited:
- EP-A- 0 696 439
- US-A- 4 399 978
- US-A- 5 280 738
- US-A- 5 871 204
- US-A- 5 913 860
- US-A- 5 913 860
- US-B2- 6 592 590

## Description

### Field of the Invention

The present invention is directed to a device for inserting, positioning and/or removing an implant from bone and, in particular, a device having a portion which can be selectively fixed or freely pivotable and/or rotatable with respect to a body portion of the device.

### Background of the Invention

Intramedullary rods or rails can be used for internal fixation and reduction of a fractured bone, such as for example a long bone. The intramedullary rod is generally inserted into the marrow canal of a long bone, such as the femur, and across the fracture so that the fracture portions are properly aligned in close apposition. The rod is typically inserted in one end of the long bone and extends into the shaft of the bone. Moreover, the rod may be designed to be backed-out of the bone to adjust the rod, remove the rod, or pull two halves of a fracture together. Various devices can aid in the insertion and/or backing-out and/or removal of the intramedullary rod.

The implant insertion and positioning device chosen by a surgeon depends on the technique the surgeon desires to use, the desired results, the implant and parameters of the operation. If a surgeon desires to freely hammer an implant into a bone and the surgical procedure so allows, a surgeon can use a hammer-type insertion device having a head and a shaft. Such a surgical hammer-type insertion device is known from US 2002/026196 A1. The head on such devices has an orientation which is fixed with respect to the shuft. A rod Inserter having an enlarged portion is attached to the intramedullary rod. The surgeon repeatedly strikes the enlarged portion of the rod inserter with the head of the hammer-type insertion device to drive the rod into the marrow canal.

On the other hand, if a surgeon desires or the surgical procedure so requires and/or allows, the surgeon can use a hammer-type insertion device where the head pivots about the shaft. Such a surgical hammer-type insertion device is known from US 5,913,860 A. This hammer-type insertion device may be used with a rod inserter, which is attached to the rod and consists of an elongated shaft having proximal and distal ends. The shaft may also have an enlarged portion at its proximal end, which is closest to the surgeon, and/or an enlarged portion at its distal end, the end closest to, and typically attached to the intramedullary rod. The hammer-type device is positioned on the shaft and travels along the shaft as it undergoes a reciprocating motion that allows a surgeon to repeatedly strike either enlarged, portion. A pivotable head allows for ease of movement as the device is reciprocated along the shaft. To drive a rod into the bone, the surgeon strikes the enlarged portion at the distal end of the shaft with the head of the hammer-type device. To back-out or remove a rod from the bone, an operator strikes the enlarged portion at the proximal end of the shaft.

### Summary of the Invention

The present invention relates to a device for impacting or inserting an implant into bone, and/or backing-out, extracting or removing the implant from the bone, and/or repositioning an implant in bone. In particular, the device can be used to insert a rod into a bone, back-out and/or remove a rod from a bone, or reposition a rod in a bone. The device comprises a shaft, a head, optionally a channel through the head, and a locking mechanism. The locking mechanism may engage the head so that the head does not pivot and/or rotate relative to the shaft. In addition, the locking mechanism may be disengaged from the head, thereby enabling the head to pivot and/or rotate freely with respect to the shaft. Preferably, the locking mechanism is associated with the shaft of the device. The device may also have means for retaining a guide in the optional channel. Additionally, the locking mechanism may have means for tightening the locking mechanism on the shaft.

In one method of impacting, repositioning or backing-out a rod from a bone, the device may be used with an implant inserter which is attached to the rod. The inserter may have an aiming arm and an enlarged portion proximate its end. One exemplary method of using the device includes the steps of: moving the locking mechanism so that it engages the head, thereby fixing the head relative to the shaft (*i.e*., the head does not pivot, rotate, or translate relative to the shaft); and striking the rod or the enlarged portion of the inserter (if an inserter is used) with the head of the device.

Another alternative method of using the device involves the steps of: attaching an implant inserter, which comprises a guide to an implant (such as, for example, an intramedullary rod); adjusting the locking mechanism so that it is disengaged from the head, thereby allowing the head to pivot or rotate relative to the shaft; positioning the device onto the guide; and reciprocating the device along the guide. The inserter used with this method may have an enlarged portion at one end of the guide proximate the rod and/or an enlarged portion at the other end of the guide. An operator can strike the rod or the enlarged portion proximate the rod to insert the rod into the bone. Alternatively, an operator can strike the enlarged portion proximate an operator to back-out, remove or reposition the rod from the bone.

In another embodiment, the head of the device may also contain a slot, which communicates with the channel of the head, preferably intersecting the channel at an angle, most preferably perpendicular to the channel. The head can be positioned on a guide by moving the slot of the head onto the guide shaft until the shaft reaches the channel. The device is then rotated to align the longitudinal axis of the guide shaft with the longitudinal axis of the channel. In yet another embodiment, the head may have a helical slot, which intersects the channel of the head. The helical slot is inserted onto the guide shaft and, as the helical slot is advanced onto the guide shaft, the head threads, rotates or twists until the longitudinal axis of the guide is aligned with the longitudinal axis of the channel.

### Brief Description of the Drawings

The present invention can be better understood by reference to the following drawings, wherein like references numerals represent like elements. The drawings are merely exemplary to illustrate certain features that may be used singularly or in combination with other features and the present invention should not be limited to the embodiments shown.

**FIG. 1** is a perspective view of the implant insertion, removal, positioning device according to the present invention;

**FIG. 2** is a side view of the device as shown in **FIG. 1**;

**FIG. 3** is a rear view of an alternative embodiment of the device according to the present invention;

**FIG. 4** is a cross-sectional view of the head of the device as shown in **FIGS. 1** and 2;

**FIG. 4A** is a cross-sectional view of a portion of an exemplary holding member;

**FIG. 4B** is a cross-sectional view of a portion of another exemplary holding member;

**FIG. 4C** is a cross-sectional view of a portion of another exemplary holding member;

**FIG. 4D** is a cross-sectional view of an alternative embodiment of the head of the device as shown in **FIGS. 1** **and** **2**;

**FIG. 4E** is a perspective view of the head of the device as shown in **FIGS. 1** and 2 with an alternative exemplary holding member;

**FIG. 5** is a perspective view of an alternative embodiment of the device according to the present invention;

**FIG. 6** is a perspective view of an alternative embodiment of the head of the device according to the present invention;

**FIG. 7** illustrates a method of using the device as shown in **FIG. 1****;** and

**FIG. 8** illustrates an alternative method of using the device as shown in **FIG. 1****.**

### Detailed Description

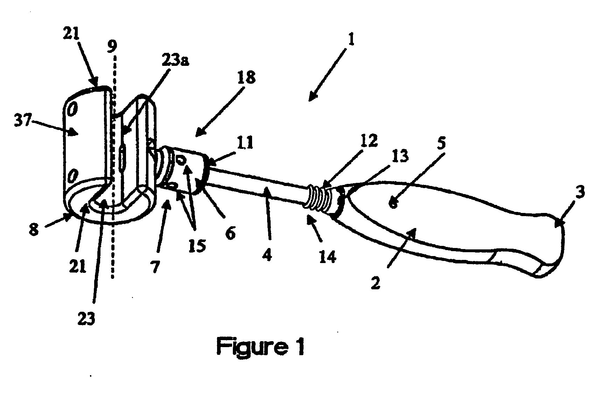

**FIG. 1** shows an exemplary embodiment of tool **1** for use in inserting, backing-out, extracting, removing and/or positioning an implant. It should, however, be understood that those of ordinary skill in the art will recognize many modifications and substitutions which may be made to various elements of the present invention. Tool 1 can be used by an operator to insert an implant into, back-out or remove an implant from, and/or position or reposition an implant in a bone. For example, tool **1** may be used to insert an intermedullary rod or nail into or back-out/remove a rod from the medullary canal of a bone. As illustrated in **FIG. 1****,** exemplary tool **1** may include a handle **2,** a shaft **4,** a locking mechanism **7** (including a locking member **6**), and an impacting body or head **8** pivotally connected to shaft 4. It should be noted that the term pivot and rotate is used interchangeably herein.

Handle **2** permits an operator to grab and hold tool **1** during use. Handle **2** is preferably ergonomically contoured for use by the operator and may contain grip enhancing surfaces, materials and/or projections. It should be noted that handle **2** is not necessary to the functioning of tool **1** since an operator can grab shaft **4** instead. Handle **2** may be made of plastic, rubber, metal, wood, a composite material (*i.e*., made of at least two materials), or any other suitable material. For example, handle **2** may be made of an impregnated linen or a phenolic resin. Alternatively, handle **2** can be made from a molded polymer (*e.g*., Delrin®). Handle **2** may also have a core material surrounded by another material. For instance, an aluminum core may be surrounding by a silicon jacket. Various factors can be considered when determining the material used to make handle **2,** including ability to withstand sterilization/cleaning (*e.g*., using an autoclave; cleaning products used for sterilization in hospitals), feel (*e.g*., a molded material gives a softer feel in a operator's hand), weight, durability (*e.g*., ability to withstand impact/load forces, ability to be dropped), resistance to staining (*e.g*., from blood or substances used in surgery or to clean tool 1), the ability to withstand heat, and the ability to grip the handle, particularly with latex gloves which are generally used during surgery. Other factors, not listed, may also be relevant.

The handle **2** can be any shape or size. For example, it may be desirable for handle **2** to be shaped to fit the contours of a hand (*i.e*., ergonomic). Moreover, the end 3 of handle **2** can be flared to prevent handle **2** from slipping from an operator's hand during use. Additionally, to allow for enhanced gripping of handle **2** by an operator, handle **2** may have a grip (not shown), which covers or is positioned in a least a portion of handle **2.**

The grip may be made out of the same materials as handle **2.** The grip may have indents or protrusions. And, the factors used to select the material for handle **2** are also applicable to choosing material(s) for the grip. The grip may be a separate piece from handle **2,** for example, the grip may be positioned as an inset (not shown) in the handle. The grip may also be over-molded (*i.e*., formed by molding a material over handle **2).** A grip made of rubber may be used. Alternatively, the grip may be integral or monolithic with handle 2 (*i.e*., the handle and grip are made of one piece of material). In such a configuration, the grip portion of handle **2** can be textured.

Shaft **4** can be attached to handle **2** or be one integral piece with handle **2.** Shaft 4 can be made from, for example, a hardened material such as stainless steel. However, shaft **4** may be made of any suitable material. Various factors may be used to determine the material of shaft **4,** including ability to withstand sterilization/cleaning, weight, durability, resistance to staining, and heat treatable. Other factors, not listed, may also be relevant. A stainless steel shaft **4** can have a diameter between about approximately **5** mm and about approximately 20 mm, preferably between about approximately 6 mm and about approximately 15 mm, and most preferably between about approximately 7 mm and about approximately 10 mm. It should be understood that the diameter of shaft **4** depends on the material used and can be any diameter so long as the diameter of shaft **4** is sufficient enough so that shaft **4** will not break or bend from repetitive use of tool 1. Moreover, shaft **4** may have a length measured from handle **2** to head **8** between about approximately 20 mm and about approximately 150 mm, preferably between about approximately 50 mm and about approximately 140 mm, and most preferably between about approximately 100 mm and about approximately 130 mm. The length of shaft 4 is one factor, amongst others, enabling an operator to properly swing tool **1,** and ensuring proper leverage and momentum of tool 1 for hammering. Furthermore, as shown in **FIG. 1****,** shaft **4** can be straight, however, any other shape (*e.g*., S-shape, L-shape, curved), which allows an operator to hammer an implant and enables the proper functioning of the locking mechanism **7** is envisioned.

Shaft **4** may abut handle **2** or extend through a portion or the entire length of handle **2.** Shaft **4** can be attached to handle **2** in numerous ways, including friction fitting, welding, chemical or molecular bonding, or gluing. In another embodiment, shaft **4** can have an external thread (not shown) engaging an internal thread (not shown) of handle **2.** In an alternative embodiment, handle **2** may have an opening **5** which receives a pin, screw, set screw, rod, bar or other retaining means which attaches shaft **4** to lock handle **2** and shaft **4** relative to each other.

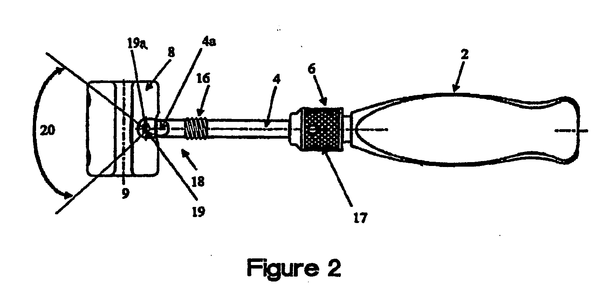

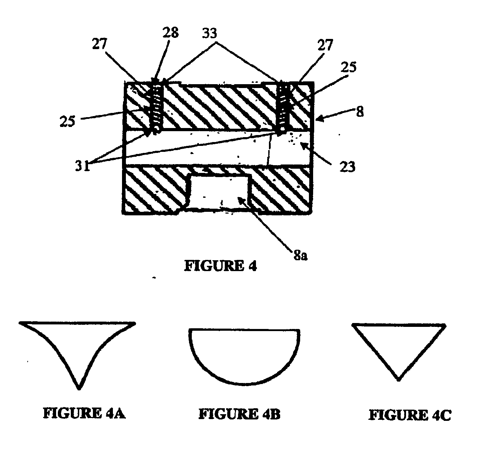

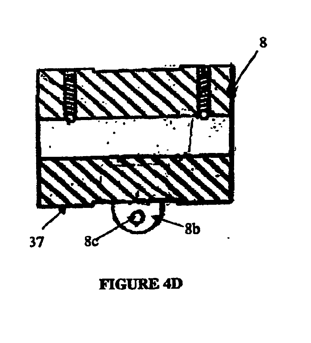

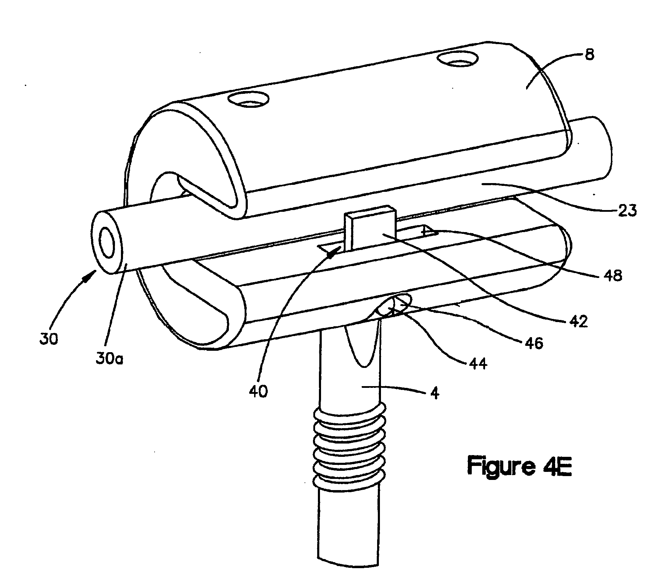

Head **8** is connected to shaft **4.** Head **8** may be preferably pivotally and/or rotationally connected to shaft **4.** In one embodiment, as shown in **FIG. 2****,** a portion 4a of distal end **18** of shaft **4** may be flattened or have a reduced diameter with a hole **19** therethrough. Reduced shaft portion **4a** may be inserted into a open area 8a (**FIG. 4**) formed in the head **8.** A pin **19a** can be positioned through hole **19** such that shaft **4** pivots or rotates about pin **19a.** The pin **19a** can be fixed with respect to head **8** by, for example, force/press fitting, welding, chemically or molecularly bonding, or gluing into receiving portion(s) (not shown) of head **8.** Alternatively, a pin can be fixed to shaft **4** (or shaft 4 may have protrusions (not shown) extending from opposite side of shaft **4).** The pin or protrusions can be positioned in receiving portion(s) (not shown) of head **8** such that head **8** can pivot or rotate with respect to shaft 4. In this manner head **8** can pivot about shaft **4.**

It should be understood that open area **8a** is not required. For example, head **8** may have an extension 8b (**FIG. 4D**) (one or more extensions **8b** may be used) extending from outer surface **37** of head **8.** The extension **8b** can have hole **8c** to receive a pin **19a.** The hole **19** in shaft **4** can be aligned with hole **8c** in extensions **8b**. Pin **19a** may be inserted through hole 8c in extension **8b** and hole **19** in shaft 4. In such a configuration, the angle **20** through which shaft **4** can swing or pivot (hereinafter referred to as the "angle of pivot") is constrained by shaft **4** contacting head **8.**

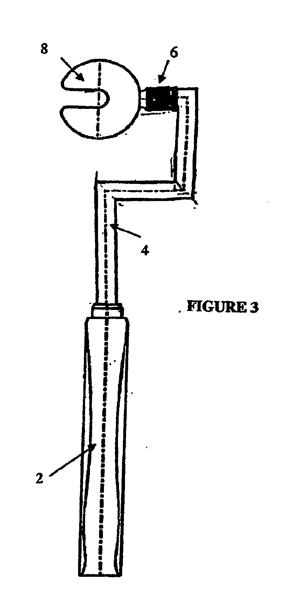

Shaft **4** and head **8** can be configured so that head **8** has an angle **20** of pivot of about 360° (*e.g*., where the shaft **4** is attached to the side of head **8** (**FIG. 3**)) but more preferably the angle **20** of pivot can be between about approximately 30° and about approximately 270° degrees, and even more preferably between about approximately 30° and about approximately 180° degrees, and most preferably between about approximately 30° and about approximately 90° degrees. The angle **20** through which shaft **4** pivots with respect to the head **8** depends upon the clearance of shaft 4 in open area 8a in the head 8. The angle **20** of pivot preferably would allow for ergonomic swinging of tool **1.** Some factors which contribute to determining an appropriate angle **20** of pivot include the length of the shaft/handle and the amount of throw that is required to generate the energy necessary for inserting an implant.

In another embodiment, shaft **4** may have a ball (not shown) at distal end **18** and head **8** may have a socket (not shown) to receive the ball of shaft **4.** Alternatively, shaft **4** and head **8** may be connected by a swivel joint (not shown) so that head 8 can be rotated about shaft **4.** Any other means of connecting shaft **4** and head **8** can also be used in a manner so that head **8** can pivot and/or rotate with respect to shaft **4** in at least one direction or about at least one axis.

A locking mechanism **7** may be provided to fix shaft **4** and head **8** with respect to each other, thereby preventing pivotal or rotational movement of head **8** about shaft 4. It should be understood that a locking mechanism includes any mechanism which functions to lock shaft **4** and head **8** relative to each other and which also allows the shaft **4** to be unlocked so that shaft **4** and head **8** can pivot with respect to each other. The locking mechanism may be associated with, or may be attached to shaft **4,** or may be attachable to shaft **4** (*i.e.,* capable of being attached/detached from shaft **4**). The locking mechanism can consist of a single piece or more than one piece.

**FIGS. 1** and **2** illustrate an exemplary locking mechanism **7.** The locking mechanism of **FIGS. 1** and **2** includes a locking member **6** disposed about shaft 4 **(****FIGS. 1** and **2**). Locking member **6** can be made of a hardened material, such as stainless steel, however, other materials can also be used. The same factors that are used to determine the material for shaft **4** can also be used to determine the material, shape, and dimensions of locking member 6. Other factors may also be considered such as the ability to be tightened on shaft **4** and resistance to deformation caused by impact loads from head **8.** To enable tightening of locking member **6** on shaft **4,** member **6** should be shaped and of sufficient size to allow an operator to grip locking member **6** with his/her fingers or, as discussed in greater detail below, with a tool or to position a tool into locking member 6. It should be understood that locking member **6** can be any shape, size, or thickness so long as it can perform its function of keeping shaft **4** and head **8** fixed relative to each other. In one exemplary embodiment, not shown, locking member **6** can be a nut.

It should be noted that the portion of locking member **6** that engages the handle **2** or head 8 can be configured to assist in holding the locking member **6** against the handle **2** or help in fixing the head **8** relative to shaft **4.** For example, locking member **6** can have a flat portion for engaging a corresponding flat portion of handle **2** and/or head **8.** Locking member **6** may also have a textured portion, for example teeth, which engages a corresponding textured or smooth portion, for example teeth, on handle **2** and/or head **8.**

Locking member 6 may have internal threads (not shown) to engage proximal threads **12** or distal threads **16.** Threads **16** may be located proximate head **8** and function to hold locking member **6** against head **8** at distal end **18** of shaft **4.** Threads **12** function to hold locking member **6** at a position on shaft **4** such that locking member **6** does not engage head 8. In **FIG. 1****,** threads **12** are located proximate handle **2** such that when locking member **6** engages threads **12,** a portion **11** of locking member **6** engages enlarged diameter portion **13** and/or handle **2.** This configuration allows for locking member **6** to be tightly fastened to the proximal end **14** of shaft **4.**

By threading locking member 6 on proximal threads 12 or by tightly fastening locking member 6 against an enlarged portion, locking member 6 is restrained from moving as the operator uses the insertion, positioning, removing tool **1**. More specifically, locking member **6** will not move along shaft **4** as the device is swung by an operator. Locking member **6** can be tighten on shaft **4** by manually twisting member **6** on threads **12** or **16** (*i.e.,* an operator uses his/her fingers/hand). Member **6** may also have a grip (*e.g*., diamond knurl **17** as shown in **FIG. 2****)** which is integral with member **6** and can take various forms, including a rough surface, grooves, or depressions on a portion or around the entire periphery of locking member **6.** In another embodiment, locking member **6** can have a grip made from a separate piece of material. A grip can be positioned around the outer surface of member **6** or can be inlaid into member **6.** For example, locking member **6** may be over-molded with a rubber jacket. Over-molded material may be made from any material that provides a better gripping surface for an operator (before, during, or after the operation) than a smooth surface.

In addition, to allow for further tightening, locking member may **6** have one or more opening **15,** which can be any shape or size, positioned about the periphery of member 6. A device such as a spanner or pin wrench, rod, or bar (not shown) can be positioned in opening **15** and used to twist locking member **6** on shaft **4.** The spanner or pin wrench, rod, bar, or other device provides an operator with leverage to further tighten locking member **6** on threads **12, 16.** It should be understood that locking member **6** may be tightened only manually or, alternatively, there can be no manual tightening and the entire tightening step can be performed using a device such as a spanner or pin wrench, rod, bar or other device (not shown), or the member **6** can be manually tightened by the operator turning the member **6** and then further tightened using a tightening tool. In addition to or in place of using devices such as the spanner or pin wrench, rod, bar, or other device, an operator may use, for example, a plier, wrench, or similar device to grab the outer surface of locking member 6 and twist member **6** on threads **12** and **16.** Moreover, to prevent member **6** from loosening on threads **12** and **16,** a screw, set screw, bolt, pin, or other retaining member can be positioned to extend through member **6** and contact the surface and/or extend into threads **12, 16** and/or shaft **4**.

In an alterative embodiment, threads **12** may be located at any location along the length of shaft **4.** To lock locking member **6** tightly to shaft **4,** a portion of threads **12** may be deformed and/or enlarged portion **13** may be positioned adjacent threads **14.** In yet another embodiment, there may be only one threaded portion on shaft **4.** The threaded portion preferably would cover enough of shaft **4** so that locking member 6 can be moved from a position adjacent head **8** to a position where member 6 disengages head **8.** In still another embodiment, the entire shaft **4** may be threaded. In another embodiment, the shaft **4** may taper from a smaller diameter at distal end **18** to a larger diameter at proximal end **14** or have an enlarged portion. Locking member **6** can be force fitted over a portion of the taper or enlarged portion to hold it on shaft **4** at a sufficient distance from head **8** so that head **8** can pivot about shaft 4. Also, in any embodiment, locking member **6,** in its disengaged position, may be moveable along shaft **4** (*i.e.,* not locked to shaft **4)** or may be designed to be removed all together from shaft **4** upon disengagement from head 8. The locking mechanism **7** may not comprise any threads at all.

In an alternative embodiment, a pin, screw, set screw, bolt, or other retaining means, can extend through member **6** and into or through shaft **4.** The pin, screw, set screw, bolt or other retaining means may extend through opening **15** or some other opening that forms a through hole. The through hole may be threaded to engage corresponding threads on the pin, screw, set screw, bolt, or other retaining means. This configuration can be used to hold member **6** on shaft **4** anywhere along the length of shaft **4.**

In yet another alternative embodiment, the locking mechanism may incorporate a bayonet fitting. For example, there can be one or more protrusions (not shown) around the periphery of shaft **4.** The protrusion(s) may be a single prong, or may extend a portion or the entire length of shaft **4.** Locking member **6** of this embodiment may have internal groove(s) along its length to receive the protrusion(s). Such a configuration allows for axial, but not rotational, movement of member **6** on shaft **4** where member **6** engage the protrusion(s). A pin, screw, set screw, bolt or other retaining means may be used to hold member **6** against head **8** or at any position along the length of shaft **4.** Alternatively, a spring (not shown) can be positioned around shaft **4** and bias locking member **6** towards head **8.** It should be appreciated that any combination of the embodiments and features disclosed herein can be used as a locking mechanism.

In an alternative embodiment, there may be no locking member 6. Such an embodiment may use a pin, set screw, screw, rod, bar, bolt or other means to fix shaft **4** so that shaft **4** does not pivot with respect to head **8.** One of ordinary skill in the art can readily appreciate that other means of temporarily fixing shaft **4** and head **8** relative to each other is envisioned by the present invention.

As shown in **FIG. 3****,** in an alternative embodiment, shaft **4** may be connected on the side of head **8.** Shaft 4 may be shaped similar to a question mark and have a handle **2.** The connection of shaft **4** and head 8 should allow for pivotal and/or rotational movement of head 8 relative to shaft **4.** It should be noted, however, that shaft **4** can be connected anywhere on the outer surface **37** (**FIG. 1**) of head **8** preferably so that head **8** can pivot and/or rotate with respect to shaft **4**, and tool 1 can be used to hammer an implant into, reposition, or remove/back-out an implant from a bone.

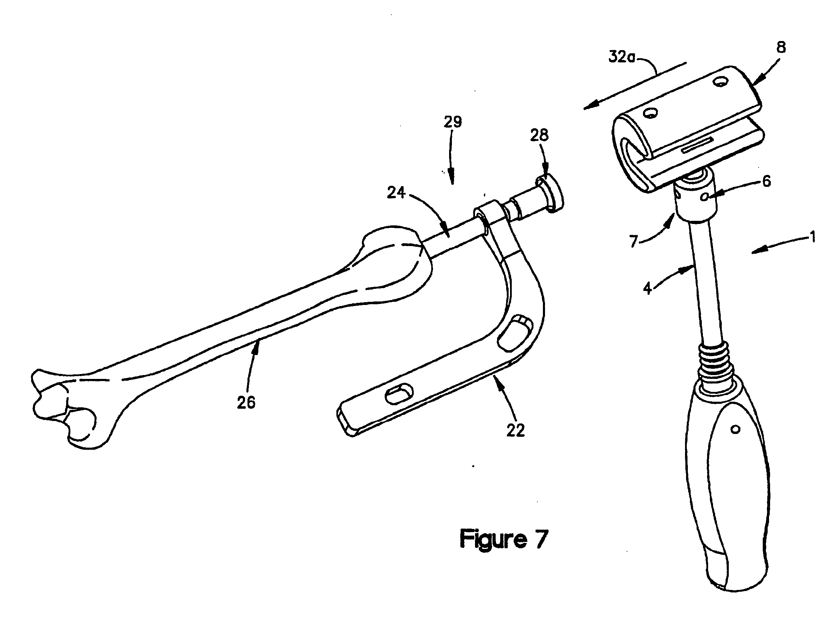

Head **8** can be any shape or size so long as head **8** can function to impact/strike an implant and/or portion of an insertion device, such as inserter **29** (**FIG. 7**) in order to impart a force to insert the implant into, reposition, or back-out the implant from a bone. Any portion of outer surface **37,** but preferably faces **21, 21a** of head **8,** can be used for impacting/striking an implant or insertion device. Additionally, faces **21, 21a** of head **8** may have a piece of material attached thereto which is made of a stronger material than head **8** (*e.g*., a material which is more resistant to dents and/or deformation). For example, a piece of titanium may be welded to face **21** and/or **21a.** In another embodiment, a softer material may be attached to faces **21, 21a** to absorb the impact force from hammering an implant. For instance, a piece of rubber can be bonded to face **21** and/or **21a.** It should be noted that a separate piece of material can be attached to any portion of head **8** that may contact another surface during hammering.

Head **8** may be made of stainless steel, but other materials can also be used. Any of the various factors discussed above with regard to choosing a material for shaft 4 or member 6 can be used to determine the material of head **8.** In addition, other factors that may be considered is resistance to dents from hammering and impact strength. Moreover, head **8** can have a length between about approximately 30 mm and about approximately 150 mm, preferably between about approximately 40 mm and about approximately 100 mm, and most preferably between about approximately 50 mm and about approximately 75 mm. And, head **8** can have a diameter of between about approximately 30 mm and about approximately 100 mm, preferably between about approximately 35 mm and about approximately 75 mm, and most preferably between about approximately 38 mm and about approximately 60 mm. Various factors can be considered when determining the dimensions of head **8,** including the weight and/or mass of the head **8** which is necessary for generating the energy needed to insert an implant.

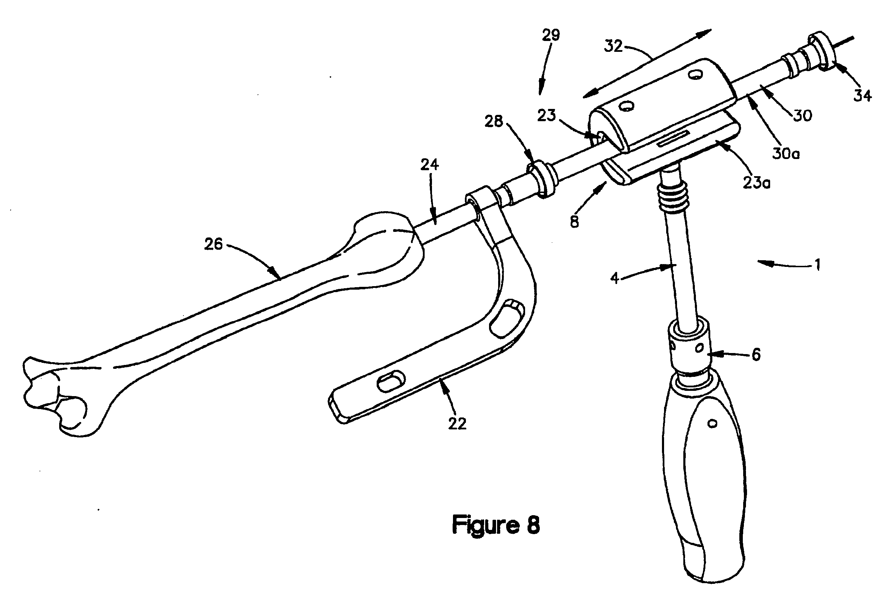

Head **8** may also have a channel **23** and a slot **23a** intersecting the channel **23.** Head 8 and channel **23** may share a longitudinal axis **9,** however, head 8 and channel **23** may each have a separate axis, which can be parallel or at any angle with respect to each other. Slot **23a** communicates with channel **23** and outer surface **37** of head **8,** thereby providing a path for inserting a guide **30,** such as longitudinal member **30a** shown in **FIG.** 8, into channel **23.** Slot **23a** may run the length of head **8** and intersect channel **23** along its entire length as shown in **FIG. 1****.** Channel **23** is sized and configured to receive guide **30** of an implant inserter, such as inserter **29** shown in **FIG. 7****.** Channel **23** can be any size so long as guide **30** can be inserted therein and channel **23** allows for reciprocation of head **8** along the guide **30.** For example, the diameter/thickness of channel **23** can be between about approximately **5** mm and about approximately **35** mm, preferably between about approximately 6 mm and about approximately **24** mm, and most preferably between about approximately 7 mm and about approximately **20** mm. When a guide **30** is positioned in channel **23,** it may be desirable to provide a mechanism or means to retain the guide **30** in channel **23** during use. **FIG. 4** shows a cross-sectional view of head **8** with an exemplary retaining member, ball plunger **25.**

Head **8** may have one or more ball plungers **25.** Ball plunger **25** may have a housing **28** containing spring **27** and ball bearing **31.** The housing is configured to prevent ball bearing **31** from falling out of the housing while, at the same time, allowing a portion of ball bearing **31** to protrude from the housing into channel **23.** Ball bearing **31** may also be held in the housing by being fixed to spring **27.** Ball bearing **31** is permitted to move up and down in the housing in response to a force thereon, for example, by insertion of rod **30** into channel **23.** Ball plunger **25** may be positioned in hole **33** (or an opening) of head **8.** Plunger **25** can have external threads (not shown) to engage threads (not shown) in the perimeter of hole **33,** thus keeping plunger **25** in place. Alternatively, plunger **25** may be connected to head **8** in various ways, including force fitting, welding, chemical or molecular bonding, or gluing. The position of plunger **25** in channel **23** or slot **23a** (*i.e.,* the extent to which plunger **25** extends into channel **23** or slot **23a)** depends on the guide that is used and the size of the channel **23**. It should be understood that ball bearing **31** can be any shape, such as for example a sphere, sloped triangular **(****FIG. 4A****),** hemi-spherical **(****FIG. 4B****),** straight sided, or triangle **(****FIG. 4C****).** The shape of ball bearing **31** should allow the guide **30** to move in and out of channel **23** when a predetermined force is applied to remove tool **1** from the guide **30.** Once the guide **30** has passed the retaining member, the head **8** preferably is permitted to move along the guide **30.** In an alternative embodiment, the retaining member may extend a portion of the length or the entire length of the channel **23.** In another embodiment, the retaining member may be, for example, a screw, set screw, pin, bar or rod, which can be positioned into or on head **8** and/or channel **23.** One of skill in the art can appreciate that other retaining mechanisms or means of holding or retaining a guide **30** in channel **23** is envisioned so long as the guide **30** can be moved in and out of channel **23** and head **8** can move along the guide **30.** Head **8** may be also be configured such that the retaining member can be adjusted (*i.e.*, the holding member can be positioned anywhere within channel **23;** the holding member can be adjusted to extend into channel **23** to various extents).

**FIG. 4E** illustrates an alternative holding member, component **40** having finger **42.** It should be understood by those of skill in the art that component **40** can be used by itself or in conjunction with other holding members such as, for example, ball plungers **25.** A portion of component **40** (not shown) can be operatively connected to shaft 4. A pin, screw, set screw, bar or rod **44** can be positioned through an opening **46** in head **8,** though an opening (not shown) in component **40,** and/or into a receiving portion (not shown) within head 8. It should be understood by those of skill in the art that any construction that allows component **40** to rotate or pivot within the head **8** is envisioned, including rotation or pivot within slot **48** as shaft **4** is rotated or pivoted. Alternatively, the component **40** may be designed so that the finger **42** moves independently of shaft **4.** Component **40** can be configured such that finger **42** can be extended (partially or entirely) into channel **23** and/or retracted (partially or entirely) into slot **48** and out of channel **23.** For example, component **40** can be designed so that it moves straight up and down, into and out of channel **23** through slot **48.** However, even in this up and down configuration, component **40** can be attached to shaft **4.**

To position the head **8** on a guide **30,** such as longitudinal member 30a, component **40** (by itself, by rotating/pivoting component **40** along with shaft **4,** or by moving component **40** straight up and down with shaft **4)** can be rotated, pivoted, or moved straight down into slot **48.** Finger **42** can be entirely positioned within the slot **48** so that no portion of finger **42** is within the channel **23.** However, finger **42** only needs to be positioned as far into slot **48** as necessary so that guide **30** can enter channel **23.** It is desirable that when head **8** is positioned on a guide **30,** such as longitudinal member **30a,** and reciprocated thereon, the component **40** does not rotate or pivot to such a degree that the head **8** becomes disengage from the guide **30.**

Moreover, head **8** can have multiple holes **33** (or opening) to allow an operator to adjust the position of the holding member, such as plunger **25,** in head **8.** Also, head **8** and plunger **25** can be configured to allow an operator to adjust the extent to which ball bearing **31** extends into channel **23.** It should be noted that any mechanism that can be used to temporarily retain a guide **30,** such as longitudinal member **30a,** in channel **23** is envisioned.

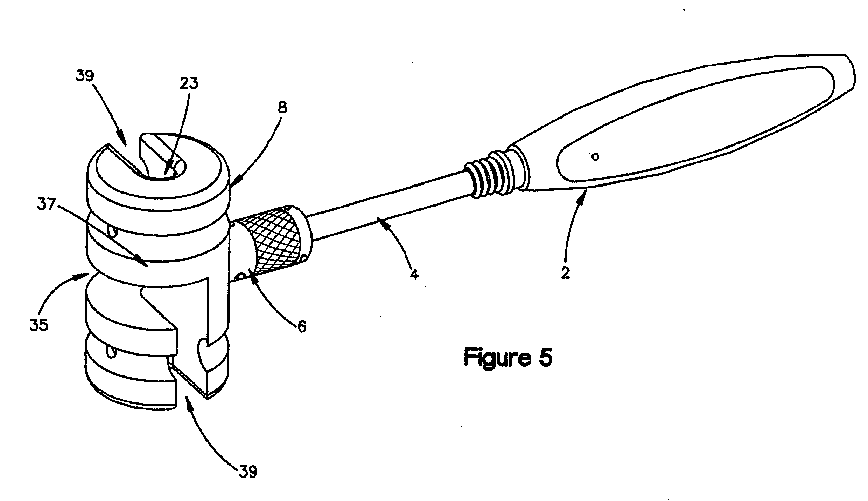

Turning now to **FIG. 5****,** illustrated is an alternative embodiment of head 8. In this embodiment, a guide **30,** such as longitudinal member **30a,** of an implant inserter, such as inserter **29,** can be twisted into channel **23.** A slot **35** can extend between outer surface **37** of head **8** and communicate with and intersect channel **23.** The axis of slot **35** may be transverse to, or more preferably oriented at a 90° angle relative to the longitudinal axis **9** of channel **23.** It should be understood that any angle between about approximately 0° and about approximately 180° is also envisioned. Furthermore, channel **23** may include opposing side channels **39,** which communicate with channel **23** and outer surface **37** of head **8,** thereby forming paths for inserting the guide **30,** in channel **23.** The slot **35** in head **8** is positioned onto the guide **30** and then rotated through side channels **39** and into channel **23,** such that the longitudinal axis of the guide **30** is aligned with the longitudinal axis **9** of channel **23.** In another alternative embodiment, there can be a helical slot that extends from the outer surface of head **8** to channel **23.**

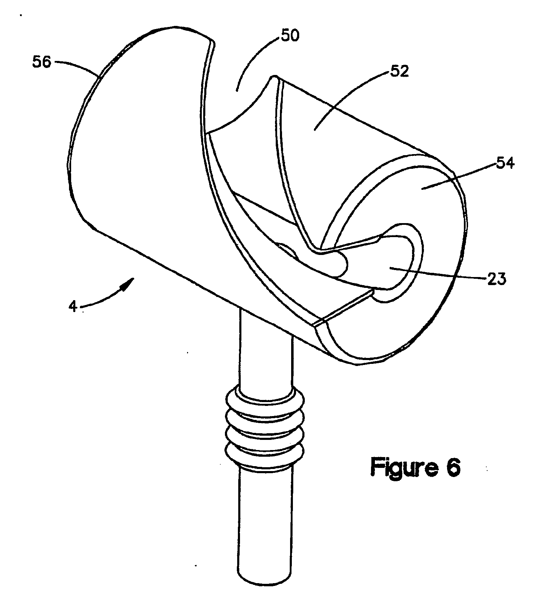

**FIG. 6** shows another alternative embodiment of head **8.** In this embodiment, head **8** can have a helical slot **50.** Head **8** can be threaded, rotated or twisted onto a guide **30,** such as longitudinal member **30a,** of an implant inserter, such as inserter **29.** The helical slot **50** can extend between outer surface **52** of head **8** and communicate with and intersect channel **23.** Helical slot **50** can also intersect front face **54** and back face **56** of outer surface **52.** As helical slot **50** in head **4** is advanced over guide **30,** head **4** will thread, twist or rotate into channel **23,** such that the longitudinal axis of guide **30** is aligned with the longitudinal axis **9** of channel **23.**

**FIGS. 7** and **8** illustrate methods of using tool **1** with a implant inserter device, such as inserter **29,** to insert, back-out, remove, position or reposition an implant, such as intramedullary rod **24,** from bone **26.** **FIG. 7** shows the use of tool **1** to insert an implant, such as rod **24,** into bone **26.** To use tool **1** as shown in **FIG. 7****,** head **8** is preferably fixed with respect to shaft **4** so that head **8** cannot pivot or rotate about shaft **4.** A locking mechanism **7,** such as locking member **6,** can be used to fix head 8 relative to shaft 4. Head **8** is fixed by threading locking member **6** on threads **16** (**FIG. 2**) at distal end **18.** Locking member **6** is tightened as described above so that it firmly engages head **8.** It should be understood that any locking mechanism can be used to fix head **8** with respect to shaft **4** so long as head **8** does not pivot and/or rotate on shaft **4.** In this configuration, the head **8** is in a "fixed position."

In the fixed position, head **8** can be used to strike or hammer an implant, such as intramedullary rod **24,** into bone **26.** To assist in hammering rod **24** into bone **26,** an implant inserter, such as inserter **29,** may be provided. Tool **1**, however, can be used to hammer the implant directly. Inserter **29** is attached to rod **24** and includes a mushroom-shaped head portion **28** and an aiming arm **22.** In use, an operator and/or an assistant holds aiming arm **22** by hand and the operator and/or the assistant strikes portion **28** with head **8** of tool **1.** By swinging tool **1** in direction **32a** and striking portion **28,** rod **24** is driven in bone **26.** It should be understood that head **8** preferably may be fixed with respect to shaft 4 when tool **1** is used with an inserter **29** shown in **FIG. 7** or with no inserter at all. It is also contemplated that head **8** may pivot with respect to shaft **4** when used with inserter **29** shown in **FIG. 6** or without any inserter at all.

Alternatively, **FIG. 8** illustrates the use of tool **1** to insert, back-out, remove, position or reposition an implant, such as rod **24,** from bone **26.** To use tool **1** as shown in **FIG. 8****,** head **8** preferably should be permitted to pivot with respect to shaft **4.** To accomplish this, the locking mechanism **7,** such as locking member **6,** is disengaged from head **8** by loosening member 6 from threads **16** and moving locking member **6** towards proximal end **14** of shaft **4.** Locking member **6** may be kept stationary on shaft **4,** so that it does not move during swinging of tool **1,** by threading locking member **6** on threads **12** (**FIG. 1**) at proximal end **14.** The locking member **6** is now spaced a distance from head **8** sufficient enough to allow head **8** to pivot with respect to shaft 4 in a direction shown by arrow **20** (**FIG. 2**). As discussed above, depending on how shaft **4** and head **8** are attached, head **8** may be able to rotate about shaft **4.** In this configuration, the head **6** is in a "toggle position" and can act similar to a slaphammer or slide hammer.

In the toggle position, head **8** may be used with an implant inserter device, such as inserter **29,** to hammer an implant, such as rod **24,** into and/or out of bone **26.** Inserter **29** is attached to rod **24** and includes an aiming arm **22,** a distal mushroom-shaped head portion **28,** a guide **30** (*e.g*., a longitudinal member **30a**,), and a proximal mushroom-shaped head portion **34.** One of ordinary skill in the art would appreciate that guide 30 may be any shape, including round, oval, square, rectangular, or other polygon, and can have any thickness so long as head **8** can fit over guide **30** and move therealong. Head **8** of tool 1 is positioned on guide **30** by moving slot **23a** over guide **30** until guide **30** enters channel **23.** Guide **30** may be retained in channel **23** using a holding member as described above. To insert rod **24** into bone **26,** an operator and/or an assistant holds arm **22** by hand and the operator and/or the assistant reciprocates head **8** on guide **30** in direction **32,** repeatedly striking portion **28** with head **8** of tool **1**. In this manner, rod **24** is driven in bone **26.** To back-out or remove rod **24** from bone **26** or reduce a fracture (*i.e*., pull two bone fragment together) using rod **24,** or reposition rod **24** in the bone, the operator and/or the assistant reciprocates head **8** on guide 30 in direction **32,** repeatedly striking portion **34** with head **8** of tool 1.

The tool of **FIG. 5** can be used by using the same reciprocating motion as described above. Also as discussed above, a locking mechanism, such as locking member **6,** can be used to fix head **8** relative to shaft **4** and can be positioned to allow head **8** to toggle with respect to shaft **4.** Only the method of positioning head **8** onto guide **30** differs. Slot **35** is positioned onto the guide **30** until the guide **30** intersects channel **23.** At this point, the longitudinal axis of the guide **30** is at an angle with the longitudinal axis of channel **23.** Head **8** is then rotated such that the guide **30** passes through opposing side channels **39** and into channel **23.** At this point, the longitudinal axis of the guide **30** aligns with the longitudinal axis **9** of channel **23.** The same mechanism and structures used to temporarily retain the guide **30** in head 8 **of** **FIGS. 1** and **2****,** such as for example plunger **25,** can be used to temporarily retain the guide **30** in the embodiment of head **8** illustrated in **FIG. 5****.** In yet another embodiment, shown in **FIG. 6****,** a guide **30,** such as longitudinal member **30a,** can be positioned in a helical slot **50.** As the helical slot 50 is advanced over the guide **30,** the head **8** threads, rotates or twists such that guide **30** is positioned in channel **23.** At this point, the longitudinal axis of the guide **30** aligns with the longitudinal axis **9** of channel **23.**

While the foregoing description and drawings represent the preferred embodiments of the present invention, it will be understood that various additions, modifications and substitutions may be made therein without departing from the scope of the present invention as defined in the accompanying claims. In particular, it will be clear to those skilled in the art that the present invention may be embodied in other specific forms, structures, arrangements, proportions, and with other elements, materials, and components, without departing from the essential characteristics thereof. One skilled in the art will appreciate that the invention may be used with many modifications of structure, arrangement, proportions, materials, and components and otherwise, used in the practice of the invention, which are particularly adapted to specific environments and operative requirements without departing from the principles of the present invention. The presently disclosed embodiments are therefore to be considered in all respects as illustrative and not restrictive, the scope of the invention being indicated by the appended claims, and not limited to the foregoing description.

## Claims

1. A surgical hammer-type insertion device (1) for use with a bone implant comprising:
an impacting body (8); a channel (23) through the impacting body (8), the channel (23) having a longitudinal axis; a shaft having a proximal end and distal end, the distal end of the shaft being operatively connected to the impacting body such that the shaft is permitted to pivot with respect to the impacting body; **characterized i**n
a locking mechanism having at least two operating positions, a first position wherein the locking mechanism permits the shaft to pivot with respect to the impacting body and a second position wherein the locking mechanism prevents the shaft from pivoting with respect to the impacting body.

2. The surgical hammer-type insertion device of claim 1, wherein the impacting body has an axis and the longitudinal axis of the channel is parallel to the axis of the impacting body.

3. The surgical hammer-type insertion device of claim 2, wherein the impacting body has an outer surface and the channel intersects the outer surface of the impacting body.

4. The surgical hammer-type insertion device of claim 3, wherein the impacting body comprises a slot communicating with the channel and the outer surface of the impacting body.

5. The surgical hammer-type insertion device of claim 4, wherein the slot is at an angle relative to the longitudinal axis of the channel.

6. The surgical hammer-type insertion device of claim 4, wherein the slot is parallel to the channel.

7. The surgical hammer-type insertion device of claim 1, further comprising a handle.

8. The surgical hammer-type insertion device of claim 1, wherein at least a portion of the shaft is threaded.

9. The surgical hammer-type insertion device of claim 8, wherein the shaft has threads proximate the impacting body and threads proximate the proximal end of the shaft and an unthreaded portion therebetween.

10. The surgical hammer-type insertion device of claim 9, wherein the locking mechanism comprises a locking member associated with the shaft, the locking member moveable to a fixed position on the shaft such that the locking member is fixed with respect to the shaft and the shaft can pivot with respect to the impacting body.

11. The surgical hammer-type insertion device of claim 8, wherein the locking mechanism has an internal thread that engages the thread portion of the shaft.

12. The surgical hammer-type insertion device of claim 1, wherein the locking mechanism in the first position disengages from the impacting body and in the second position engages the impacting body.

13. The surgical hammer-type insertion device of claim 1, wherein the locking mechanism comprises means for tightening the locking mechanism on the shaft, wherein preferably the means for tightening comprises a portion for receiving a tool.

14. The surgical hammer-type insertion device of claim 1, further comprising side channels, the side channels communicating with the channel, the outer surface of the head and the slot.

15. The surgical hammer-type insertion device of claim 1, wherein the slot is a helical slot intersecting the channel and the outer surface of the head.

16. The surgical hammer-type insertion device of claim 1, wherein at least a portion of the distal end of the shaft comprises threads, wherein preferably the locking mechanism is configured and designed to engage the threads on at least a portion of the distal end of the shaft, the locking mechanism contacting the head when engaging the distal threads to prevent movement of the head relative to the shaft.

17. The surgical hammer-type insertion device of claim 1, wherein at least a portion of the proximal end of the shaft comprises threads, wherein preferably the locking mechanism is configured and designed to engage the threads on the at least a portion of the proximal end of the shaft, the locking mechanism positioned a distance from the head when engaging the proximal threads to permit movement of the head relative to the shaft.

18. The surgical hammer-type insertion device of claim 1, wherein the locking member in the first position disengages from the head.

19. The surgical hammer-type insertion device of claim 1, further comprises means for retaining a guide in the channel.

20. The surgical hammer-type insertion device of claim 19, wherein the means for retaining is a plunger, the plunger comprising a spring and bearing in the head, the bearing being capable of movement in the channel to retain the guide in the channel.

21. The surgical hammer-type insertion device of claim 19, wherein the means for retaining is a finger, the finger being moveable within the channel.

22. The surgical hammer-type insertion device of claim 19, wherein the means for retaining defines a first diameter in the channel for receiving the guide, the means for retaining being adjustable such that the means for retaining defines a second diameter in the channel for receiving the guide.

## Patentansprüche

1. Einführvorrichtung (1) in der Art eines chirurgischen Hammers zur Verwendung mit einem Knochenimplantat, umfassend: einen Schlagkörper (8), eine Auskehlung (23) durch den Schlagkörper (8), wobei die Auskehlung (23) eine Längsachse aufweist; einen Schaft mit einem proximalen und einem distalen Ende, wobei das distale Ende von dem Schaft funktionell mit dem Schlagkörper derart verbunden ist, dass der Schaft gegenüber dem Schlagkörper geschwenkt werden kann; **gekennzeichnet durch**
einen Verriegelungsmechanismus, der mindestens zwei Arbeitsstellungen aufweist, eine erste Position, in welcher der Verriegelungsmechanismus es zulässt, dass der Schaft gegenüber dem Schlagkörper geschwenkt wird und eine zweite Position, in welcher der Verriegelungsmechanismus es verhindert, dass der Schaft gegenüber dem Schlagkörper geschwenkt wird.

2. Einführvorrichtung in der Art eines chirurgischen Hammers nach Anspruch 1, wobei der Schlagkörper eine Achse aufweist und die Längsachse von der Auskehlung parallel zu der Achse des Schlagkörpers ist.

3. Einführvorrichtung in der Art eines chirurgischen Hammers nach Anspruch 2, wobei der Schlagkörper eine Außenfläche aufweist und die Auskehlung die Außenfläche des Schlagkörpers durchschneidet.

4. Einführvorrichtung in der Art eines chirurgischen Hammers nach Anspruch 3, wobei der Schlagkörper einen Schlitz aufweist, der mit der Auskehlung und der Außenfläche des Schlagkörpers in Verbindung steht.

5. Einführvorrichtung in der Art eines chirurgischen Hammers nach Anspruch 4, wobei der Schlitz bezüglich der Längsachse von der Auskehlung in einem Winkel angeordnet ist.

6. Einführvorrichtung in der Art eines chirurgischen Hammers nach Anspruch 4, wobei der Schlitz parallel zu der Auskehlung angeordnet ist.

7. Einführvorrichtung in der Art eines chirurgischen Hammers nach Anspruch 1, die ferner ein Griffstück aufweist.

8. Einführvorrichtung in der Art eines chirurgischen Hammers nach Anspruch 1, wobei mindestens ein Teilbereich von dem Schaft mit einem Gewinde versehen ist.

9. Einführvorrichtung in der Art eines chirurgischen Hammers nach Anspruch 8, wobei der Schaft ein Gewinde unmittelbar neben dem Schlagkörper und ein Gewinde unmittelbar am proximalen Ende des Schaftes und dazwischen einen Teilbereich ohne Gewinde aufweist.

10. Einführvorrichtung in der Art eines chirurgischen Hammers nach Anspruch 9, wobei der Verriegelungsmechanismus ein Verriegelungselement enthält, das mit dem Schaft verbunden ist, wobei das Verriegelungselement zu einer festgelegten Position von dem Schaft derart verschiebbar ist, dass das Verriegelungselement bezüglich des Schaftes fixiert ist und der Schaft gegenüber dem Schlagkörper geschwenkt werden kann.

11. Einführvorrichtung in der Art eines chirurgischen Hammers nach Anspruch 8, wobei der verriegelungsmechanismus ein internes Gewinde aufweist, das in das Gewindeteil von dem Schaft eingreift.

12. Einführvorrichtung in der Art eines chirurgischen Hammers nach Anspruch 1, wobei der Verriegelungsmechanismus in der ersten Position den Schlagkörper freigibt und in der zweiten Position den Schlagkörper einrastet.

13. Einführvorrichtung in der Art eines chirurgischen Hammers nach Anspruch 1, wobei der Verriegelungsmechanismus Mittel zum Festmachen des Verriegelungsmechanismus an dem Schaft aufweist, wobei vorzugsweise das Mittel zum Festmachen einen Abschnitt für die Aufnahme von einem Werkzeug enthält.

14. Einführvorrichtung in der Art eines chirurgischen Hammers nach Anspruch 1, die ferner eine seitliche Auskehlung enthält, wobei die seitliche Auskehlung mit der Auskehlung, der Außenfläche von dem Hammerkopf und dem Schlitz in Verbindung steht.

15. Einführvorrichtung in der Art eines chirurgischen Hammers nach Anspruch 1, wobei der Schlitz ein spiralförmiger Schlitz ist, der die Auskehlung und die Außenfläche des klammerkopfes durchschneidet.

16. Einführvorrichtung in der Art eines chirurgischen Hammers nach Anspruch 1, wobei mindestens ein Teilbereich von dem distalen Ende des Schaftes ein Gewinde aufweist, wobei vorzugsweise der Verriegelungsmechanismus so konfiguriert und entworfen ist, dass er in das Gewinde in mindestens einem Bereich von dem distalen Ende des Schaftes eingreift, wobei der Verriegelungsmechanismus mit dem Hammerkopf in Kontakt ist, wenn er in das distale Gewinde eingreift, um eine Bewegung von dem Hammerkopf gegenüber dem Schaft zu verhindern.

17. Einführvorrichtung in der Art eines chirurgischen Hammers nach Anspruch 1, wobei mindestens ein Teilbereich von dem proximalen Ende des Schaftes ein Gewinde aufweist, wobei vorzugsweise der Verriegelungsmechanismus so konfiguriert und entworfen ist, dass er in das Gewinde in mindestens einem Bereich von dem proximalen Ende des Schaftes eingreift, wobei der Verriegelungsmechanismus in einem Abstand zu dem Hammerkopf positioniert ist, wenn er in das proximale Gewinde eingreift, um eine Bewegung von dem Hammerkopf gegenüber dem Schaft zu ermöglichen.

18. Einführvorrichtung in der Art eines chirurgischen Hammers nach Anspruch 1, wobei das Verriegelungselement in der ersten Position dem Hammerkopf freigibt.

19. Einführvorrichtung in der Art eines chirurgischen Hammers nach Anspruch 1, die ferner ein Mittel zum Festhalten eines Führungselements in der Auskehlung aufweist.

20. Einführvorrichtung in der Art eines chirurgischen Hammers nach Anspruch 1, wobei das Mittel zum Festhalten ein Stößel ist, der Stößel eine Feder und ein Lager in seinem Kopf aufweist, das Lager in der Auskehlung bewegt werden kann, um das Führungselement in der Auskehlung zu halten.

21. Einführvorrichtung in der Art eines chirurgischen Hammers nach Anspruch 19, wobei das Mittel zum Festhalten ein Haltefinger ist und der Haltefinger innerhalb der Auskehlung beweglich ist.

22. Einführvorrichtung in der Art eines chirurgischen Hammers nach Anspruch 19, wobei das Mittel zum Festhalten einen ersten Durchmesser in der Auskehlung für die Aufnahme des Führungselements definiert, wobei das Mittel zum Festhalten derart verstellbar ist, dass das Mittel zum Festhalten einen zweiten Durchmesser in der Auskehlung für die Aufnahme des Führungselements definiert.

## Revendications

1. Dispositif d'insertion de type marteau chirurgical (1) destiné à être utilisé avec un implant osseux, qui comprend :
un corps d'impact (8) ; un canal (23) dans le corps d'impact (8), le canal (23) ayant un axe longitudinal ; un arbre ayant une extrémité proximale et une extrémité distale, l'extrémité distale de l'arbre étant connectée de manière opérationnelle au corps d'impact de manière à ce que l'arbre puisse pivoter relativement au corps d'impact ; **caractérisé en ce que**
un mécanisme de verrouillage ayant au moins deux positions de fonctionnement, une première position dans laquelle le mécanisme de verrouillage permet à l'arbre de pivoter relativement au corps d'impact et une seconde position dans laquelle le mécanisme de verrouillage empêche le pivotement de l'arbre relativement au corps d'impact.

2. Dispositif d'insertion de type marteau chirurgical selon la revendication 1, dans lequel le corps d'impact a un axe et l'axe longitudinal du canal est parallèle à l'axe du corps d'impact.

3. Dispositif d'insertion de type marteau chirurgical selon la revendication 2, dans lequel le corps d'impact a une surface extérieure et le canal coupe la surface extérieure du corps d'impact.

4. Dispositif d'insertion de type marteau chirurgical selon la revendication 3, dans lequel le corps d'impact comprend une fente communiquant avec le canal et la surface extérieure du corps d'impact.

5. Dispositif d'insertion de type marteau chirurgical selon la revendication 4, dans lequel la fente se situe à un angle relativement à l'axe longitudinal du canal.

6. Dispositif d'insertion de type marteau chirurgical selon la revendication 4, dans lequel la fente est parallèle au canal.

7. Dispositif d'insertion de type marteau chirurgical selon la revendication 1, comprenant en outre une poignée.

8. Dispositif d'insertion de type marteau chirurgical selon la revendication 1, dans lequel au moins une partie de l'arbre est filetée.

9. Dispositif d'insertion de type marteau chirurgical selon la revendication 8, dans lequel l'arbre possède un filetage à proximité du corps d'impact et un filetage à proximité de l'extrémité proximale de l'arbre et une partie non filetée entre ceux-ci.

10. Dispositif d'insertion de type marteau chirurgical selon la revendication 9, dans lequel le mécanisme de verrouillage comprend un élément de verrouillage associé à l'arbre, l'élément de verrouillage pouvant être mis dans une position fixe sur l'arbre de manière à ce que l'élément de verrouillage soit fixe relativement à l'arbre et à ce que l'arbre puisse pivoter relativement au corps d'impact.

11. Dispositif d'insertion de type marteau chirurgical selon la revendication 8, dans lequel le mécanisme de verrouillage a un filetage interne qui se met en prise avec la partie filetée de l'arbre.

12. Dispositif d'insertion de type marteau chirurgical selon la revendication 1, dans lequel le mécanisme de verrouillage dans la première position se libère du corps d'impact et dans la seconde position se met en prise avec le corps d'impact.

13. Dispositif d'insertion de type marteau chirurgical selon la revendication 1, dans lequel le mécanisme de verrouillage comprend un moyen pour serrer le mécanisme de verrouillage sur l'arbre, dans lequel de préférence le moyen de serrage comprend une partie destinée à recevoir un outil.

14. Dispositif d'insertion de type marteau chirurgical selon la revendication 1, comprenant en outre des canaux latéraux, les canaux latéraux communiquant avec le canal, la surface extérieure de la tête et la fente.

15. Dispositif d'insertion de type marteau chirurgical selon la revendication 1, dans lequel la fente est une fente hélicoïdale coupant le canal et la surface extérieure de la tête.

16. Dispositif d'insertion de type marteau chirurgical selon la revendication 1, dans lequel au moins une partie de l'extrémité distale de l'arbre comprend un filetage, dans lequel de préférence le mécanisme de verrouillage est configuré et conçu pour se mettre en prise avec le filetage sur au moins une partie de l'extrémité distale de l'arbre, le mécanisme de verrouillage contactant la tête lorsqu'il est en prise avec le filetage distal pour empêcher le mouvement de la tête relativement à l'arbre.

17. Dispositif d'insertion de type marteau chirurgical selon la revendication 1, dans lequel au moins une partie de l'extrémité proximale de l'arbre comprend un filetage, dans lequel de préférence le mécanisme de verrouillage est configuré et conçu pour se mettre en prise avec le filetage sur une ou plusieurs parties de l'extrémité proximale de l'arbre, le mécanisme de verrouillage étant positionné à distance de la tête lorsqu'il est en prise avec le filetage proximal pour permettre le mouvement de la tête relativement à l'arbre.

18. Dispositif d'insertion de type marteau chirurgical selon la revendication 1, dans lequel l'élément de verrouillage dans la première position se libère de la tête.

19. Dispositif d'insertion de type marteau chirurgical selon la revendication 1, comprenant en outre un moyen pour retenir un guide dans le canal.

20. Dispositif d'insertion de type marteau chirurgical selon la revendication 19, dans lequel le moyen de retenue est un plongeur, le plongeur comprenant un ressort et un roulement dans la tête, le roulement étant capable de mouvement dans le canal pour retenir le guide dans le canal.

21. Dispositif d'insertion de type marteau chirurgical selon la revendication 19, dans lequel le moyen de retenue est un doigt, le doigt étant amovible à l'intérieur du canal.

22. Dispositif d'insertion de type marteau chirurgical selon la revendication 19, dans lequel le moyen de retenue définit un premier diamètre dans le canal pour recevoir le guide, le moyen de retenue étant ajustable de manière à ce que le moyen de retenue définisse un second diamètre dans le canal pour recevoir le guide.
